# EUROPEAN PATENT APPLICATION

(11) **EP 4 755 377 A1**
(43) Date of publication of application: **10.06.2026**
(21) Application number: 25158794.5
(22) Date of filing: 19.02.2025
(51) Int. Cl.: A61K 9/70, A61K 31/465

(54) **TRANSDERMAL PATCH**

(30) Priority: 04.12.2024 TW 113146890
(71) Applicant: Teh Seng Pharmaceutical MFG, CO., LTD., Tainan City 710 (TW)
(72) Inventor: HSU, Shih-Pin, 710 Tainan City (TW)
(74) Representative: van Dam, Vincent

(57) **Abstract**

A transdermal patch is provided, which comprises: a backing layer; a first drug-containing adhesive layer comprising a first nicotine or a pharmaceutically acceptable salt thereof and a first adhesive; a second drug-containing adhesive layer comprising a second nicotine or a pharmaceutically acceptable salt thereof and a second adhesive; and a release liner, wherein the first drug-containing adhesive layer and the second drug-containing adhesive layer is disposed between the backing layer and the release liner.

## Description

### CROSS-REFERENCE TO RELATED APPLICATIONS

This application claims the benefits of the Taiwan Patent Application Serial Number 113146890, filed on December 4, 2024, the subject matter of which is incorporated herein by reference.

### BACKGROUND OF THE INVENTION

### Field

The present invention relates to a transdermal patch and, more specifically a transdermal patch comprising nicotine or a pharmaceutically acceptable salt thereof.

### Description of Related Art

According to statistical reports, approximately one fifth of the world's population will suffer from cancer, and due to the aging of the population, the proportion of cancer is increasing year by year. Lung cancer has no obvious symptoms in the early stages, and its symptoms are similar to those of various lung diseases and difficult to distinguish. Therefore, it has often progressed to the late stages when it is discovered. According to the World Health Organization, the number of new cancer cases and mortality rates from lung cancer have long ranked among the top three. In addition, data show that at least 22% of lung cancer is caused by smoking, including smokers themselves, living with smokers and exposure to secondhand smoke. Therefore, quitting smoking can be considered one of the most direct ways to prevent lung cancer.

However, according to statistics, only about 3% of people can successfully quit smoking by their own willpower alone, while using nicotine replacement therapy to quit smoking can increase the success rate by about 40%-60% compared to quitting smoking by willpower alone. The smoking cessation patch is one of the nicotine replacement therapies, which has the advantages of simple use, stable blood concentration and/or good concealment.

Therefore, nicotine patches still need to be studied and improved to provide consumers with another choice.

### SUMMARY OF THE INVENTION

The present invention provides a transdermal patch, comprising: a backing layer; a first drug-containing adhesive layer comprising a first nicotine or a pharmaceutically acceptable salt thereof and a first adhesive; a second drug-containing adhesive layer comprising a second nicotine or a pharmaceutically acceptable salt thereof and a second adhesive; and a release liner, wherein the first drug-containing adhesive layer and the second drug-containing adhesive layer are disposed between the backing layer and the release liner.

In the present invention, the backing layer may be a film, a sheet, a sheet-like porous body, a sheet-like foam, knitted fabrics, nonwoven fabrics, paper, a lamination thereof, which may be prepared by a resin selected from the group consisting of polyethylene, polypropylene, polybutadiene, ethylene-vinyl acetate copolymer, vinyl acetate-vinyl chloride copolymer, polyvinyl chloride, polyamide, polyester, nylon, cellulose derivatives, polyurethane.

In one embodiment of the present invention, a thickness of the first drug-containing adhesive layer may range between 10 µm and 500 µm, for example, between 20 µm and 400 µm, 30 µm and 300 µm or 50 µm and 150 µm, but the present invention is not limited thereto. In one embodiment of the present invention, a thickness of the second drug-containing adhesive layer may range between 10 µm and 500 µm, for example, between 20 µm and 400 µm, 30 µm and 300 µm or 50 µm and 150 µm, but the present invention is not limited thereto. In one embodiment of the present invention, a ratio of the thickness of the first drug-containing adhesive layer to the thickness of the second drug-containing adhesive layer may be 1:5 to 5:1, for example, may be 1:1, 1:2, 1:3, 1:4, 1:5, 5:1, 4:1, 3:1 or 2:1, but the present invention is not limited thereto. In one embodiment of the present invention, the ratio of the thickness of the first drug-containing adhesive layer to the thickness of the second drug-containing adhesive layer may be 1:1.

In one embodiment of the present invention, based on a total weight of the first drug-containing adhesive layer, a content of the first nicotine or the pharmaceutically acceptable salt thereof may range between 5 wt% and 30 wt%, and for example, between 10 wt% and 25 wt%, 10 wt% and 20 wt%, 12 wt% and 18 wt% or 12 wt% and 16 wt%, but the present invention is not limited thereto. In one embodiment of the present invention, based on a total weight of the second drug-containing adhesive layer, a content of the second nicotine or the pharmaceutically acceptable salt thereof may range between 5 wt% and 30 wt%, and for example, between 10 wt% and 25 wt%, 10 wt% and 20 wt%, 12 wt% and 18 wt% or 12 wt% and 16 wt%, but the present invention is not limited thereto.

In one embodiment of the present invention, based on a total weight of the first drug-containing adhesive layer, a content of the first adhesive may range between 50 wt% and 94.9 wt% and, for example, between 60 wt% and 90 wt%, 70 wt% and 90 wt% or 80 wt% and 90 wt%, but the present invention is not limited thereto. In one embodiment of the present invention, based on a total weight of the second drug-containing adhesive layer, a content of the second adhesive may range between 50 wt% and 94.9 wt% and, for example, between 60 wt% and 90 wt%, 70 wt% and 90 wt%, 80 wt% and 90wt% or 80 wt% and 94.9 wt%, but the present invention is not limited thereto.

In one embodiment of the present invention, the first adhesive and the second adhesive may respectively comprise a pressure sensitive adhesive. In one embodiment of the present invention, the pressure sensitive adhesive may be an acrylic-based pressure sensitive adhesive. In one embodiment of the present invention, the acrylic-based pressure sensitive adhesive may be selected from the group consisting of Duro-Tak^{®} 87-2353, Duro-Tak^{®} 387-2353, Duro-Tak^{®} 87-900A, Duro-Tak^{®} 87-9301, Duro-Tak^{®} 87-4098, Duro-Tak^{®} 87-2510, Duro-Tak^{®} 387-2510, Duro-Tak^{®} 87-2287, Duro-Tak^{®} 387-2287, Duro-Tak^{®} 87-4287, Duro-Tak^{®} 87-2516, Duro-Tak^{®} 387-2516, Duro-Tak^{®} 87-2074, Duro-Tak^{®} 87-235A, Duro-Tak^{®} 87-2852, Duro-Tak^{®} 87-2051, Duro-Tak^{®} 387-2051, Duro-Tak^{®} 87-2052, Duro-Tak^{®} 387-2052, Duro-Tak^{®} 87-2054, Duro-Tak^{®} 387-2054, Duro-Tak^{®} 87-2194, Duro-Tak^{®} 87-2196 and a combination thereof.

In one embodiment of the present invention, the first drug-containing adhesive layer and the second drug-containing adhesive layer may selectively further comprise an excipient. In one embodiment of the present invention, the first drug-containing adhesive layer may comprise a first excipient, and based on a total weight of the first drug-containing adhesive layer, a content of the first excipient may range between 0.1 wt% and 1 wt% and, for example, between 0.1 wt% and 0.8 wt% or 0.1 wt% and 0.5 wt%, but the present invention is not limited thereto. In one embodiment of the present invention, the second drug-containing adhesive layer may comprise a second excipient, and based on a total weight of the second drug-containing adhesive layer, a content of the second excipient may be between 0.1 wt% and 1 wt% and, for example, between 0.1 wt% and 0.8 wt% or 0.1 wt% and 0.5 wt%, but the present invention is not limited thereto.

In one embodiment of the present invention, the excipient may be, for example, an antioxidant. Suitable antioxidant may be, for example, vitamin E, vitamin E derivatives, ascorbic acid, ascorbic acid derivatives, isoascorbic acid, isoascorbic acid derivatives, gallic acid derivatives, 2,6-butylhydroxytoluene (BHT), butylated hydroxyanisole (BHA), tributylhydroquinone (TBHQ), 2-mercaptobenzimidazole or a combination thereof, but the present invention is not limited thereto.

In the present invention, the release liner may be a silicon-coated release liner (a silicon-coated liner), but the present invention is not limited thereto.

In one embodiment of the present invention, the transdermal patch may not contain a semi-permeable paper or a semi-permeable membrane. The "semi-permeable paper or semi-permeable membrane" refers to a paper or film that has the ability to control the drug release rate. In one embodiment of the present invention, the transdermal patch may not comprise a rate-controlling adhesive, such as polyisobutylene (PIB), propylene glycol caprylate, polyglyceryl-3 dioleate, other rate-controlling adhesive or a combination thereof, but the present invention is not limited thereto.

The present invention further provides a method for manufacturing a transdermal patch, which comprises the following steps: forming a first drug-containing adhesive layer on a backing layer and forming a second drug-containing adhesive layer on a release liner; and adhering the first drug-containing adhesive layer and the second drug-containing adhesive layer, wherein the first drug-containing adhesive layer and the second drug-containing adhesive layer are disposed between the backing layer and the release liner, wherein the first drug-containing adhesive layer comprises a first nicotine or a pharmaceutically acceptable salt thereof and a first adhesive, the second drug-containing adhesive layer comprises a second nicotine or a pharmaceutically acceptable salt thereof and a second adhesive.

In one embodiment of the present invention, before the step of forming the first drug-containing adhesive layer on the backing layer, the method may further comprise: forming the first drug-containing adhesive layer on another release liner; and adhering the first drug-containing adhesive layer on the release liner and the backing layer, wherein the first drug-containing adhesive layer is disposed between the backing layer and the release liner. In one embodiment of the present invention, before the step of forming the first drug-containing adhesive layer on the release liner, the method may further comprise: mixing the first nicotine or the pharmaceutically acceptable salt thereof, the first adhesive, the first excipient and a solvent to form a first mixed glue. The first mixed glue may be applied on the release liner through a coating process to form the first drug-containing adhesive layer on the release liner. Similarly, before the step of forming the second drug-containing adhesive layer on the release liner, the method may further comprise: mixing the second nicotine or the pharmaceutically acceptable salt thereof, the second adhesive, the second excipient and a solvent to form a second mixed glue. The second mixed glue may be applied on the release liner through a coating process to form the second drug-containing adhesive layer on the release liner.

In the present invention, suitable coating processes may comprise: spin coating, roller coating, doctor blade coating, spray coating or a combination thereof, but the present invention is not limited thereto. In the present invention, the "solvent" may include, for example, chloroform, dichloromethane or ethyl acetate, but the present invention is not limited thereto.

In one embodiment of the present invention, before the step of adhering the first drug-containing adhesive layer on the release liner and the backing layer, the method may further comprise: performing a baking step on the first drug-containing adhesive layer on the release liner. In one embodiment of the present invention, after forming the second drug-containing adhesive layer on the release liner, the method may further comprise: performing a baking step on the second drug-containing adhesive layer on the release liner. The "baking step" refers to, for example, heating in an oven at a predetermined temperature for a predetermined time, the purpose of which is to remove the solvent in the adhesive layer. Therefore, the predetermined temperature and the predetermined time can be adjusted according to the situation.

In one embodiment of the present invention, before the step of adhering the first drug-containing adhesive layer and the second drug-containing adhesive layer, the method may further comprise a step of: removing the release liner on the first drug-containing adhesive layer, so that the first drug-containing adhesive layer and the second drug-containing adhesive layer can be face-to-face adhered.

The present invention is designed to enable the transdermal patch to achieve the effect of controlling the drug release rate without using semi-permeable paper, semi-permeable membrane or rate-controlling adhesive, thereby saving costs.

Other novel features of the present invention will become more apparent from the following detailed description when taken in conjunction with the accompanying drawings.

### BRIEF DESCRIPTION OF DRAWINGS

FIG. 1 shows the *in vitro* skin permeation test results of the transdermal patches of Examples 1 to 5 and Comparative Examples 1 to 5.

FIG. 2 is the drug solubility test results of the transdermal patches of Example 1 to Example 5.

### DETAILED DESCRIPTION OF THE INVENTION

The following is an explanation of the embodiments of the present invention by means of specific embodiments. A person skilled in the art can easily understand other advantages and effects of the present invention from the contents disclosed in this specification. The present invention may also be implemented or applied through other different specific embodiments, and the details in this specification may be modified and changed in various ways according to different viewpoints and applications without departing from the spirit of the present invention.

The present invention will be described in more detail by way of examples, but these examples are not intended to limit the scope of the present invention. Unless otherwise specified, the "%" used to express the content of components and the amount of substances in the following examples and comparative examples are based on weight.

The conventional chemical formula of nicotine is C₁₀H₁₄N₂, and can be represented by the following structural formula:

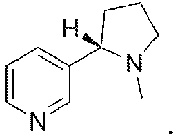

### Method for manufacturing a transdermal patch

### Examples

According to the contents shown in Table 1, nicotine, acrylic-based pressure sensitive adhesive (Duro-Tak^{®} 87-9301), vitamin E and ethyl acetate (about 4-6 times the amount of nicotine) were placed in a blender. The mixture was stirred at a rotation speed of 1500 rpm for about 10 minutes to form a first mixed glue and a second mixed glue.

The first mixed glue was coated on the first release liner by a coating machine, and the coating speed was about 0.5±0.1 m/min. The first release liner with the first mixed glue was placed in an oven and baked at a temperature of 70 °C to 120 °C to remove the solvent in the first mixed glue to form a first drug-containing adhesive layer. Next, the first release liner with the first drug-containing adhesive layer was laminated to the backing layer, wherein the first drug-containing adhesive layer was disposed between the first release liner and the backing layer.

The above coating steps were repeated to coat the second mixed glue on the second release liner at a coating speed of about 0.5±0.1 m/min. The second release liner with the second mixed glue was placed in an oven and baked at a temperature of 70 °C to 120 °C to remove the solvent in the second mixed glue to form a second drug-containing adhesive layer.

Then, the backing layer with the first drug-containing adhesive layer was separated from the first release liner. Next, the backing layer with the first drug-containing adhesive layer and the second release liner with the second drug-containing adhesive layer were laminated to each other so that the first drug-containing adhesive layer and the second drug-containing adhesive layer were located between the backing layer and the second release liner, thus forming the transdermal patch of the present invention. The final coating thickness is shown in Table 1, and the total coating weight is about 14.28 mg/cm². The transdermal patch can be further punched into the required size as needed, wherein the subsequent tests were conducted using patches of 30 cm² each.

**Table 1**

| | Example 1 | Example 2 | Example 3 | Example 4 | Example 5 |
|---|---|---|---|---|---|
| First nicotine | 15 wt% | 14 wt% | 13 wt% | 12.5 wt% | 12.25 wt% |
| First adhesive | 84.8 wt% | 85.8 wt% | 86.8 wt% | 87.3 wt% | 87.55 wt% |
| Vitamin E | 0.2 wt% | 0.2 wt% | 0.2 wt% | 0.2 wt% | 0.2 wt% |
| Thickness | 100 µm | 100 µm | 100 µm | 100 µm | 100 µm |
| Second nicotine | 15 wt% | 14 wt% | 13 wt% | 12.5 wt% | 12.25 wt% |
| Second adhesive | 84.8 wt% | 85.8 wt% | 86.8 wt% | 87.3 wt% | 87.55 wt% |
| Vitamin E | 0.2 wt% | 0.2 wt% | 0.2 wt% | 0.2 wt% | 0.2 wt% |
| Thickness | 100 µm | 100 µm | 100 µm | 100 µm | 100 µm |

### Comparative examples

According to the contents shown in Table 2, nicotine, acrylic-based pressure sensitive adhesive (Duro-Tak^{®} 87-9301), vitamin E, other additives and ethyl acetate (about 4-6 times the amount of nicotine) were placed in a blender. The mixture was stirred at a rotation speed of 1500 rpm for about 10 minutes to form a mixed glue.

The mixed glue was coated on the release liner by a coating machine, and the coating speed was about 0.5±0.1 m/min. The release liner with the mixed glue was placed in an oven and baked at a temperature of 70 °C to 120 °C to remove the solvent in the mixed glue to form a drug-containing adhesive layer. Next, the release liner with the drug-containing adhesive layer was laminated to the backing layer, wherein the drug-containing adhesive layer was disposed between the release liner and the backing layer to form the transdermal patch of Comparative examples. The final coating thickness is shown in Table 2, and the total coating weight is about 14.28 mg/cm². The transdermal patch can be further punched into the required size as needed, wherein the subsequent tests were conducted using patches of 30 cm² each.

**Table 2**

| | Comparative example 1 | Comparative example 2 | Comparative example 3 | Comparative example 4 | Comparative example 5 |
|---|---|---|---|---|---|
| Nicotine | 18 wt% | 18 wt% | 18 wt% | 18 wt% | 18 wt% |
| Adhesive | 77.3 wt% | 75.8 wt% | 72.8 wt% | 75.8 wt% | 73.8 wt% |
| Propylene glycol monocaprylate | 2 wt% | 2 wt% | 3 wt% | - | - |
| Polyglycerol-3-dioleate | 2.5 wt% | 4 wt% | 6 wt% | 6 wt% | 8 wt% |
| Vitamin E | 0.2 wt% | 0.2 wt% | 0.2 wt% | 0.2 wt% | 0.2 wt% |
| Thickness | 110 µm | 100 µm | 115 µm | 110 µm | 100 µm |

### Permeation test

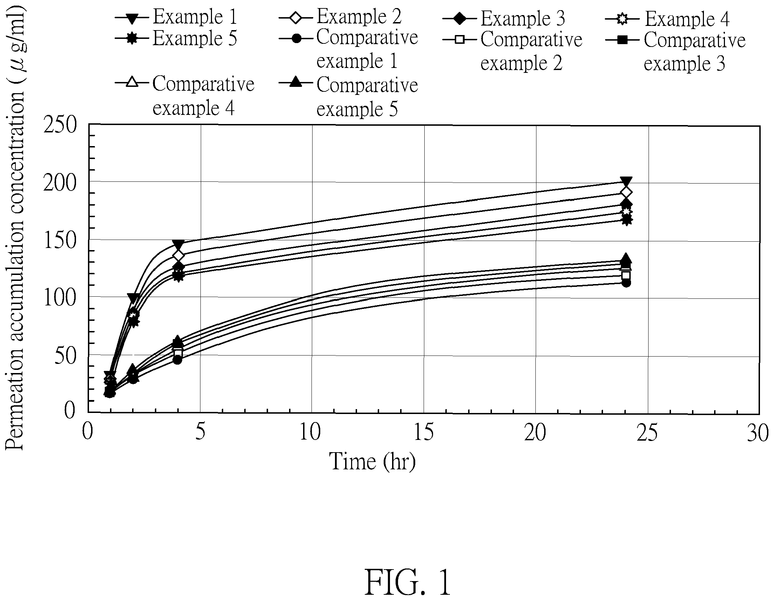

In order to measure the transdermal absorbability of nicotine in the transdermal patch, a skin permeation test was performed using the transdermal patches of Examples 1 to 5 and Comparative Examples 1 to 5. The transdermal patches of Examples 1 to 5 and Comparative Examples 1 to 5 were attached to the skin of Sprague-Dawley (SD) rats. Samples were taken at various time points (1 hour, 2 hours, 4 hours and 24 hours) to measure the accumulation content of nicotine. The results are shown in FIG. 1. As can be observed from FIG. 1, compared with the transdermal patches of Comparative Examples 1 to 5, the transdermal patches of Examples 1 to 5 have better skin permeability of nicotine.

### In vitro drug dissolution test

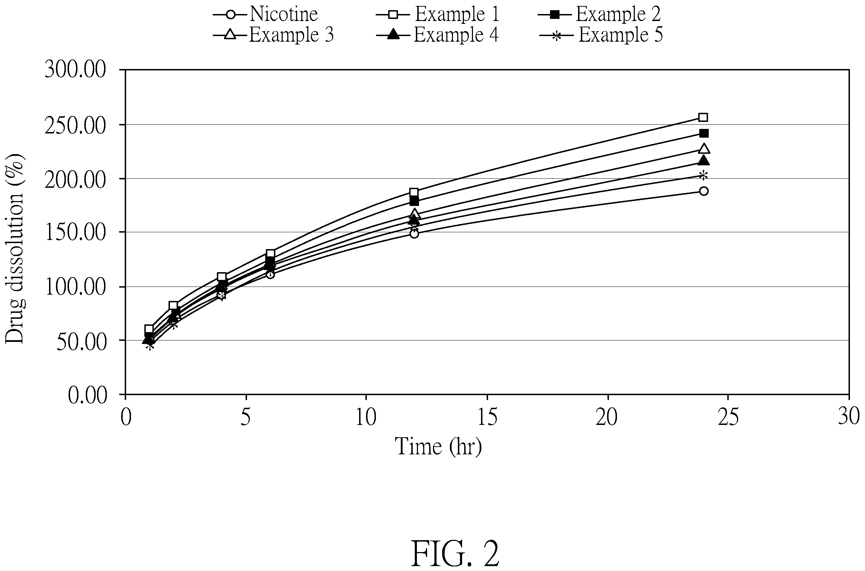

The release liners of the transdermal patches of Examples 1 to 5 were removed, and the transdermal patches were placed in a dissolution tester for dissolution testing. The paddle over disk was used, the medium was deionized water (900 mL), the rotation speed was 50 rpm, and the temperature was 32°C. Samples were taken at various time points (1 hour, 2 hours, 4 hours, 6 hours, 12 hours and 24 hours) and the solubility of the transdermal patch was measured by HPLC. Nicotine was used as a control group for comparison. The results are shown in FIG. 2. FIG. 2 shows that the transdermal patches of Examples 1 to 5 have similar dissolution rates to nicotine.

### Bioequivalence test

In order to determine that the transdermal patch of the present invention has similar bioavailability to the commercially available Nicotinell^{®}, a bioequivalence test was performed. Healthy subjects were divided into two groups, and after fasting for at least 10 hours overnight, they were given the transdermal patch of the present invention or the commercially available transdermal patch of Nicotinell^{®} for 24 hours. Blood was drawn 18 times within 36 hours (before administration, 1, 2, 3, 4, 6, 8, 10, 12, 14, 16, 20, 24, 25, 26, 28, 32, and 36 hours after administration), and nicotine concentrations in blood samples were analyzed using LC-MS/MS. The obtained pharmacokinetic parameters are shown in Table 3, and the statistical analysis results are shown in Table 4. The transdermal patch of the present invention used is the composition of Example 5, which contains 52.5 mg of nicotine; the Nicotinell^{®} used is a commercially available nicotine patch containing semi-permeable paper or semi-permeable membrane, which contains 52.5 mg of nicotine.

**Table 3**

| Pharmacokinetic parameters | Example 5 | Nicotinell^{®} |
|---|---|---|
| AUC₀₋ₜ (hr × ng/mL) | 574.680±421.571 | 499.101±280.250 |
| AUC_{0-∞} (hr × ng/mL) | 608.201±482.778 | 532.539±341.764 |
| Cₘₐₓ (ng/mL) | 28.3498±17.9956 | 24.3509±13.5445 |
| MRT (hr) | 17.9450±2.1338 | 18.4253±2.4819 |
| Tₘₐₓ (hr) | 16.84±4.21 | 18.76±4.60 |
| T_{1/2} (hr) | 4.1844±1.1651 | 4.0779±1.3937 |
| kₑₗ (1/hr) | 0.1765±0.0426 | 0.1822±0.0394 |

**Table 4**

| Statistical parameters | Geometric least square mean ratio (T/R) | 90% confidence interval (%) |
|---|---|---|
| ln (AUC₀₋ₜ) | 109.88% | 102.34-117.99 |
| ln (AUC_{0-∞}) | 109.51% | 101.87-117.73 |
| ln (Cₘₐₓ) | 112.94% | 105.14-121.31 |

| | | |
|---|---|---|
| T: Example R: Nicotinell^{®} | | |

Since the 90% confidence intervals of the parameters AUC₀₋ₜ, AUC_{0-∞} and Cₘₐₓ all met the predetermined bioequivalence acceptance limits, the bioequivalence between the two transdermal patches was demonstrated. This shows that the absorption rate and degree of the transdermal patch of the present invention are comparable to those of Nicotinell^{®}.

### Adhesion Test

In order to evaluate the adhesion strength of the transdermal patch of the present invention, the transdermal patch was subjected to an adhesion test, and commercially available Nicotinell^{®} was used as a control group for comparison. Healthy subjects were divided into two groups and received the transdermal patch of the present invention or the transdermal patch of commercially available Nicotinell^{®} for 24 hours. At each time point (0 h, 4 h, 8 h, 12 h, 16 h, 20 h and 24 h), photos were taken to observe the adhesion of the transdermal patch, and scores were given according to the adhesion. The results are shown in Table 5. The numbers in the table represent the number of individuals that meet the adhesion score at that time point, and the numbers in brackets are expressed as percentages.

**Table 5**

| | Example (n=31) | | | | | Nicotinell^{®} (n=30) | | | | |
|---|---|---|---|---|---|---|---|---|---|---|
| | Adhesion score | | | | | Adhesion score | | | | |
| Time | 0 | 1 | 2 | 3 | 4 | 0 | 1 | 2 | 3 | 4 |
| 0hr | 31 (100%) | 0 (0%) | 0 (0%) | 0 (0%) | 0 (0%) | 30 (100%) | 0 (0%) | 0 (0%) | 0 (0%) | 0 (0%) |
| 4hr | 31 (100%) | 0 (0%) | 0 (0%) | 0 (0%) | 0 (0%) | 24 (80%) | 4 (13.3%) | 2 (6.7%) | 0 (0%) | 0 (0%) |
| 8hr | 30 (96.8%) | 1 (3.2%) | 0 (0%) | 0 (0%) | 0 (0%) | 22 (73.3%) | 6 (20%) | 2 (6.7%) | 0 (0%) | 0 (0%) |
| 12hr | 30 (96.8%) | 1 (3.2%) | 0 (0%) | 0 (0%) | 0 (0%) | 22 (73.3%) | 6 (20%) | 2 (6.7%) | 0 (0%) | 0 (0%) |
| 16hr | 29 (93.5%) | 2 (6.5%) | 0 (0%) | 0 (0%) | 0 (0%) | 22 (73.3%) | 6 (20%) | 2 (6.7%) | 0 (0%) | 0 (0%) |
| 20hr | 29 (93.5%) | 1 (3.2%) | 0 (0%) | 0 (0%) | 1 (3.2%) | 22 (73.3%) | 6 (20%) | 2 (6.7%) | 0 (0%) | 0 (0%) |
| 24hr | 28 (90.9%) | 2 (6.5%) | 0 (0%) | 0 (0%) | 1 (3.2%) | 20 (66.7%) | 8 (26.7%) | 1 (3.3%) | 1 (3.3%) | 0 (0%) |

| | | | | | | | | | | |
|---|---|---|---|---|---|---|---|---|---|---|
| n: number of individuals to be tested | | | | | | | | | | |

### Adhesion score standard

0: ≥90% adhesion (basically no skin peeling)
1: ≥75% to <90% adhesion (only some edges of the patch peel away from the skin)
2: ≥50% to <75% adhesion (less than half of the patch peels from the skin)
3: > 0% to < 50% adhered but not detached (more than half of the patch peeled from the skin but did not fall off)
4: 0% adhesion (patch falls off and completely detaches from the skin)

The experimental results in Table 5 show that the transdermal patch of the present invention has good adhesion fastness and has better adhesion performance than the commercially available Nicotinell^{®} as the control group.

In summary, the transdermal patch of the present invention can achieve similar bioavailability to commercially available Nicotinell^{®} by using a double-layer adhesive layer containing drug without using semi-permeable paper, semi-permeable membrane or rate-controlling adhesive. In addition, the transdermal patch of the present invention has excellent adhesion and/or permeability.

The above specific embodiments are to be construed as merely illustrative, and not limitative of the remainder of the disclosure in any way whatsoever.

Although the present disclosure has been explained in relation to its embodiment, it is to be understood that many other possible modifications and variations can be made without departing from the spirit and scope of the disclosure as hereinafter claimed.

## Claims

1. A transdermal patch, comprising:
a backing layer;
a first drug-containing adhesive layer comprising a first nicotine or a pharmaceutically acceptable salt thereof and a first adhesive;
a second drug-containing adhesive layer comprising a second nicotine or a pharmaceutically acceptable salt thereof and a second adhesive; and
a release liner,
wherein the first drug-containing adhesive layer and the second drug-containing adhesive layer are disposed between the backing layer and the release liner.

2. The transdermal patch of claim 1, wherein a thickness of the first drug-containing adhesive layer ranges between 10 µm and 500 µm.

3. The transdermal patch of claim 1, wherein a thickness of the second drug-containing adhesive layer ranges between 10 µm and 500 µm.

4. The transdermal patch of claim 1, wherein a content of the first nicotine or the pharmaceutically acceptable salt thereof ranges between 5 wt% and 30 wt% based on a total weight of the first drug-containing adhesive layer, and a content of the second nicotine or the pharmaceutically acceptable salt thereof ranges between 5 wt% and 30 wt% based on a total weight of the second drug-containing adhesive layer.

5. The transdermal patch of claim 1, wherein the first adhesive and the second adhesive respectively comprises a pressure sensitive adhesive.

6. The transdermal patch of claim 5, wherein the pressure sensitive adhesive is an acrylic-based pressure sensitive adhesive.

7. The transdermal patch of claim 1, wherein the first drug-containing adhesive layer and the second drug-containing adhesive layer selectively further comprises an excipient.

8. The transdermal patch of claim 7, wherein the first drug-containing adhesive layer comprises a first excipient, the second drug-containing adhesive layer comprises a second excipient, a content of the first excipient ranges between 0.1 wt% and 1 wt% based on a total weight of the first drug-containing adhesive layer, and a content of the second excipient ranges between 0.1 wt% and 1 wt% based on a total weight of the second drug-containing adhesive layer.

9. The transdermal patch of claim 1, wherein the transdermal patch does not contain a semi-permeable paper or a semi-permeable membrane.

10. A method for manufacturing a transdermal patch, comprising the following steps:
forming a first drug-containing adhesive layer on a backing layer and forming a second drug-containing adhesive layer on a release liner; and
adhering the first drug-containing adhesive layer and the second drug-containing adhesive layer, wherein the first drug-containing adhesive layer and the second drug-containing adhesive layer are disposed between the backing layer and the release liner,
wherein the first drug-containing adhesive layer comprises a first nicotine or a pharmaceutically acceptable salt thereof and a first adhesive, and the second drug-containing adhesive layer comprises a second nicotine or a pharmaceutically acceptable salt thereof and a second adhesive.
